(19) Europäisches Patentamt
European Patent Office
Office européen des brevets

(11) **EP 2 305 241 A1**

(12) **EUROPEAN PATENT APPLICATION**

(43) Date of publication:
06.04.2011 Bulletin 2011/14

(21) Application number: 10012576.4

(22) Date of filing: 16.09.2005

(51) Int Cl.:
*A61K 31/198* (2006.01)  *A61K 31/7068* (2006.01)
*A61K 31/7072* (2006.01)  *A61K 31/7076* (2006.01)
*A61K 31/708* (2006.01)  *A61P 1/04* (2006.01)
*A61P 1/14* (2006.01)  *A61P 43/00* (2006.01)
*A23L 1/30* (2006.01)  *A23L 1/305* (2006.01)

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 17.09.2004 JP 2004271884

(62) Document number(s) of the earlier application(s) in
accordance with Art. 76 EPC:
09005515.3 / 2 103 306
05785845.8 / 1 806 134

(71) Applicant: Ajinomoto Co., Inc.
Tokyo 104-8315 (JP)

(72) Inventors:
• **Uneyama, Hisayuki**
**Kawasaki-shi**
**Kanagawa (JP)**
• **Tanaka, Tatsuro**
**Kawasaki-shi**
**Kanagawa (JP)**
• **Torii, Kunio**
**Kawasaki-shi**
**Kanagawa (JP)**
• **Fujita, Shinichi**
**Kawasaki-shi**
**Kanagawa (JP)**

(74) Representative: **Nicholls, Kathryn Margaret et al**
**Mewburn Ellis LLP**
**33 Gutter Lane**
**London**
**EC2V 8AS (GB)**

Remarks:
This application was filed on 30-09-2010 as a
divisional application to the application mentioned
under INID code 62.

(54) **Use of a 5'-nucleotide for preventing/improving functional digestive disorder**

(57) The present invention provides an agent and a food for the prophylaxis or improvement of a functional gastrointestinal disorder, which contains 5'-nucleotide, or a salt thereof, as an active ingredient.

FIG. 7

EP 2 305 241 A1

## Description

**Technical Field**

The present invention relates to an agent for the prophylaxis or improvement of a functional gastrointestinal disorder. More particularly, the present invention relates to an agent for the prophylaxis or improvement of functional gastrointestinal disorders (FGIDs), particularly, upper gastrointestinal dysfunctions such as functional dyspepsia (FD). (e.g., abdominal pain, heavy stomach, heartburn and the like), gastroesophageal reflux disease (GERD) and the like, a gastrointestinal motility function promoter, an agent for the prophylaxis or improvement of dysphagia and a serotonin and/or nitric oxide release promoter. The present invention also relates to a food for the prophylaxis or improvement of a functional gastrointestinal disorder and the like.

**Background Art**

Even with the advancement in endoscopic diagnosis, there are many cases where a complaint of the upper gastrointestinal symptoms such as upper abdominal pain, discomfort, postprandial heavy stomach, nausea, vomiting and the like cannot be fully explained. Such condition where a complaint of gastrointestinal symptom is reported but no organic disease is found by a general checkup including endoscopic examination, and no finding to elucidate the symptom is available is referred to as an FD (functional dyspepsia: non-ulcer dyspepsia (NUD): upper abdominal indefinite complaint). According to The American Gastroenterological Association, FD is defined to be a pathology where organic diseases such as peptic ulcer and cancer symptoms are not observed, but upper abdominal indefinite complaint continues for 4 weeks or longer, such as feeling of fullness in the abdomen, nausea vomiting, upper abdominal pain, anorexia, abnormal bowel movement and the like, based on the retention of contents in the stomach.

On the other hand, in Japan, such case has been determined to be "upper abdomen gastrointestinal complaint associated with chronic gastritis" irrespective of organic findings, and, in clinical situations, diagnosed conventionally as "gastritis" or "chronic gastritis". Currently, the subtype of FD includes an ulcer symptom type, a gastrointestinal dysmotility type and non-specific type, which include conventional gastroatonia, nervous dyspepsia and gastric neurosis.

Even in cases where an organic disease (reflux esophagitis, peptic ulcer, acute gastritis, gastrointestinal cancer, pancreas biliary disease etc.) is clearly observed, abdominal pain, discomfort, postprandial heavy stomach, nausea-vomiting and the like are also found. Accordingly, there is an urgent need for the improvement of such discomfortable feeling to ensure better QOL of patients. When NUD is joined with lower abdomen indefinite complaint such as defecation difficulty, unrelieved feeling after defecation, abdominal pain, feeling of fullness in the abdomen and the like due to constipation, about 30%-50% of the total population of Japan is assumed to have experienced some gastrointestinal indefinite complaint. The development of abdominal indefinite complaint is considered to be influenced by sex, aging, stress or overweight due to the western style diet, and is a disease representing the modern society along with the lifestyle-related diseases. Even though it is such a serious disease, the etiology of the gastrointestinal indefinite complaint is merely suggested to involve various diseases (chronic gastritis, diabetes, overweight, constipation etc.), and the only suggested mechanism of its onset is degraded gastrointestinal motility function.

In addition, many of the patients with a progressive degenerative disease of the brain such as Parkinson's disease, Huntington chorea, olivopontocerebellar atrophy and the like, cerebral apoplexy and the like also develop gastrointestinal motility dysfunction, and improvement of QOL by the improvement of gastrointestinal motility function is considered to be necessary. It is considered that many of these patients cannot report indefinite complaint by themselves due to logopathy, disturbance of consciousness and the like. Thus, a care that removes a disturbance of sensation such as indefinite complaint and the like simultaneously with a care of organic dysfunction leads to the improvement of QOL in a true sense.

5-HT4 receptor agonists and the like have heretofore been used for the treatment of FD. For example, cisapride and metoclopramide have a hyperanakinesia action on the stomach and intestines, and have been used for the treatment of the symptoms and the like of chronic gastritis, feeling of fullness in the abdomen, reflux esophagitis, abdominal indefinite complaint and pseudoileus. However, metoclopramide shows a side effect of extrapyramidal symptoms caused by the action on dopamine D2 receptors in the central nervous system, and cisapride has also been clarified to show parkinsonian symptoms. While mosapride etc. have also been used, the effect is not always sufficient, and side effects such as feeling of fullness in the abdomen and the like appear. While H2 antagonists and proton pump inhibitors have been used for the treatment of gastroesophageal reflux disease (GERD), since the safety of long-term administration has not been established, a periodic examination is necessary. Therefore, it is difficult to expect a treatment effect of these existing pharmaceutical agents while ensuring sufficient safety.

Moreover, therapeutic drugs for FD and gastrointestinal indefinite complaint accompanying a gastrointestinal organic disorder, partial 5-HT3 receptor agonist, nitroglycerol, nitric oxide (hereinafter NO)-releasing drugs such as nitrate etc. and the like are known. However, since 5-HT receptors and nitrergic proteins, which are the target of these pharmaceutical agents, are distributed in not only the gastrointestinal mucous membrane but also organs in the body including brain, the agents show various physiological activities. To be specific, when a 5-HT3 antagonist is used as an antiemetic agent, a non-specific 5-HT3 receptor agonist particularly induces nausea and vomiting. It is also known that nonspecific NO release in the systemic circulation induces low blood pressure. Therefore, it is necessary to develop a safe and highly effective pharmaceutical agent that can be acted on these targets limited in the gastrointestinal organs.

On the other hand, glutamine has been reported to show an effect of improving organic gastrointestinal diseases such as ulcer and the like without expressing a side effect (Elia M, Lunn PG., Nutrition. 1997 Jul-Aug; 13(7-8): 743-7). However, glutamine has low solubility, is highly unstable in an aqueous solution, and lacks convenience. A report has documented that addition of monosodium glutamate alone, and both glutamic acid and sodium inosinate, to diet can enhance gastric secretion in an experiment model of atrophy gastritis, which is also one of the organic diseases (Vasilevskaia LS, et al., Vopr Pitan. 1993 May-Jun; (3): 29-33, Rymshina MV & Vasilevskaia LS., Vopr Pitan. 1996; (1): 9-11). Monosodium glutamate has been confirmed to enhance gastric secretion in atrophy gastritis patients, and moreover, glutamic acid and sodium inosinate show a potential to be digestion promoters in atrophy gastritis patients (Kochetkov AM, Vopr Pitan. 1992 Sep-Dec; (5-6): 19-22, Shlygin GK, Klin Med (Mosk). 1991 Aug; 69(8): 66-70). However, a treatment effect of glutamic acid and sodium inosinate has not been suggested yet for the sensory abnormality (indefinite complaint) associated with NUD and digestive trouble.

In addition, there are reports on several attempts to improve gastrointestinal function and increase efficiency of nutrition support in patients with organic disorder in the gastrointestinal tract or patients with lower function caused thereby, by enteral administration of a glutamic acid-containing composition for the purpose of protecting the mucous membrane and improving the motility of the lower gastrointestinal tract (DE-B-4133366, US patent application publication No. 2003/138476, EP-B-0318446, JP-A-56-57385, CA-B-2404005, JP-A-48-30583). In these cases, however, maintenance of gastrointestinal barrier by protection of the mucous membrane, improvement of the motility of the lower gastrointestinal tract and the like are desired by addition of glutamic acid to a nutritional composition such as amino acid, protein and the like, and a treatment effect on FD is not suggested.

## Disclosure of the Invention

As mentioned above, 5-HT agonists and NO releasing agents have conventionally been used as therapeutic agents for FD. While serotonin and NO are systemically distributed, they are particularly abundantly present in the gastrointestinal tract. Particularly, it is considered that about 80% of serotonin is present in the gastrointestinal mucosal epithelium. There are abundant findings relating to the physiological activities of NO and serotonin in the gastrointestinal tract. NO in the gastrointestinal mucous membrane is considered to relate to receptive relaxation upon food intake, promotion of mucus secretion, repair of disordered mucous membrane, enhancement of gastrointestinal immunity, sterilization of gastrointestinal lumen, improvement of microcirculation by increased mucosal blood flow and to antiplatelet aggregation effect, and the like, and plays an important role for the maintenance of gastrointestinal function. In addition, serotonin is a main physiologically active substance of the gastrointestinal tract, which is responsible for gastrointestinal motility regulation, gastrointestinal exocrine regulation (gastric-acid secretion, pancreatic exocrine secretion etc.). When the action of these substances is artificially inhibited, the gastrointestinal function is prevented, which causes ulcer, gastrointestinal bleeding, and abnormal motility.

Therefore, a gastrointestinal indefinite complaint can be taken as a warning from the gastrointestinal tract, and it is easily assumed that a gastrointestinal indefinite complaint can be caused by a nonorganic gastrointestinal dysfunction of a mild level which is pathologically difficult to judge, not to mention an event of gastrointestinal organic pathology. Therefore, 5-HT agonists that promote serotonin release and NO releasing agents that promote NO have conventionally been used. However, these pharmaceutical agents are problematic in terms of systemic side effects and safety as mentioned above. Hence, there is a demand for the development of a pharmaceutical agent that specifically promotes release of serotonin and NO in the gastrointestinal tract and improves various conditions associated with FD and the like.

The present invention has been made in this situation and aims at providing a pharmaceutical agent or a food capable of improvement of upper gastrointestinal dysfunction such as functional gastrointestinal disorders, particularly functional dyspepsia, esophageal reflux and the like, promotion of gastrointestinal motility function, and prophylaxis or improvement of dysphagia. Moreover, the present invention aims at providing a pharmaceutical agent or a food for specifically promoting a release of serotonin and/or NO in the gastrointestinal tract.

The present inventors have conducted intensive studies in an attempt to solve the above-mentioned problems and found that when at least one kind of glutamic acid, 5'-nucleotide and a salt thereof is administered to the subject of administration, the concentration of NO and serotonin increases only in the gastrointestinal tract to promote gastrointestinal motility function, and therefore, functional gastrointestinal disorders and dysphagia can be improved without inducing systemic side effects, which resulted in the completion of the present invention.

Here, with regard to the absorbability of monosodium glutamate and 5'-nucleotide, for example, it has been reported that when monosodium glutamate and sodium inosinate are orally taken, not less than 90% of glutamic acid taken is completely oxidized on the gastrointestinal mucous membrane into carbon dioxide and water and the remaining 10% or less is converted to alanine and lactic acid, and thus, the amount of glutamic acid that transfers into blood is extremely small (PJ. Reeds, DG Burin, B Stoll, Jahoor, J. Nutrition 130: 978S (2000)). Moreover, the study of Niijima et al. affords the finding that administration of monosodium glutamate into the stomach and duodenum activates the vagus nerve afferent pathway, but intravenous and intraportal administration of monosodium glutamate does not activate the vagus nerve (Niijima A., et al., Physiol Behav. 1991 May; 49(5): 1025-8, Niijima A., et al., J Nutr. 2000 Apr; 130 (4S Suppl): 9715-3S). From these findings, it is considered that even if a part of glutamic acid is absorbed and transferred to the systemic circulation, glutamic acid does not directly activate the vagus nerve afferent pathway in the body.

From these findings, the action mechanism of improvement of functional gastrointestinal disorders by the pharmaceutical agent of the present invention is assumed to be as follows. That is, when the pharmaceutical agent of the present invention is administered to the subject of administration, release of NO and/or serotonin is promoted only in the stomach mucous membrane. As a result, the concentration of NO and/or serotonin increases only in such topical environment, and gastrointestinal motility function is promoted. Thus, the indefinite complaint associated with functional gastrointestinal disorders such as FD and the like can be improved safely and effectively. In addition, since the amount of active ingredient transferred to blood is extremely low, systemic side effects are seldom induced.

The present invention encompasses the following.

(1) An agent for the prophylaxis or improvement of a functional gastrointestinal disorder, which comprises, as an active ingredient, at least one kind selected from the group consisting of glutamic acid, 5'-nucleotide and a salt thereof.

(2) The agent of (1), wherein the aforementioned 5'-nucleotide is selected from the group consisting of 5'-inosinic acid, 5'-guanylic acid, 5'-adenyl acid, 5'-cytidylic acid, 5'-uridylic acid and 5'-xanthylic acid.

(3) The agent of (1), wherein the aforementioned 5'-nucleotide is selected from 5'-inosinic acid and 5'-guanylic acid.

(4) The agent of any one of (1) to (3), wherein the aforementioned salt is a salt with basic amino acid.

(5) The agent of (4), wherein the basic amino acid is selected from the group consisting of arginine, lysine and ornithine.

(6) The agent of (4), wherein the basic amino acid is arginine.

(7) The agent of (1), wherein the aforementioned active ingredient is an arginine salt with glutamic acid.

(8) The agent of any one of (1) to (7), wherein the aforementioned functional gastrointestinal disorder is upper gastrointestinal dysfunction.

(9) The agent of (8), wherein the aforementioned upper gastrointestinal dysfunction is functional dyspepsia or gastroesophageal reflux disease.

(10) The agent of any one of (1) to (9), wherein a daily dose of the aforementioned active ingredient to an adult is 0.01 g to 20 g.

(11) An agent for the promotion of gastrointestinal motility function, which comprises, as an active ingredient, at least one kind selected from the group consisting of glutamic acid, 5'-nucleotide and a salt thereof.

(12) An agent for the prophylaxis or improvement of dysphagia, which comprises, as an active ingredient, at least one kind selected from the group consisting of glutamic acid, 5'-nucleotide and a salt thereof.

(13) An NO and/or serotonin release promoter specific to the gastrointestinal tract, which comprises, as an active ingredient, at least one kind selected from the group consisting of glutamic acid, 5'-nucleotide and a salt thereof.

(14) The release promoter of (13), wherein the aforementioned gastrointestinal tract is stomach.

(15) A food for promoting gastrointestinal motility function, which comprises at least one kind of compound selected from the group consisting of glutamic acid, 5'-nucleotide and a salt thereof.

(16) A food for the prophylaxis or improvement of dysphagia, which comprises at least one kind of compound selected from the group consisting of glutamic acid, 5'-nucleotide and a salt thereof.

(17) A food for promoting release of NO and/or serotonin specific to the gastrointestinal tract, which comprises at least one kind of compound selected from the group consisting of glutamic acid, 5'-nucleotide and a salt thereof.

(18) The food of (17), wherein the aforementioned gastrointestinal tract is stomach.

(19) A food for the prophylaxis or improvement of a functional gastrointestinal disorder, which comprises at least one kind of compound selected from the group consisting of glutamic acid, 5'-nucleotide and a salt thereof.

(20) The food of (19), wherein the aforementioned 5'-nucleotide is selected from the group consisting of 5'-inosinic acid, 5'-guanylic acid, 5'-adenyl acid, 5'-cytidylic acid, 5'-uridylic acid and 5'-xanthylic acid.

(21) The food of (19), wherein the aforementioned 5'-nucleotide is selected from 5'-inosinic acid and 5'-guanylic acid.

(22) The food of any one of (19) to (21), wherein the aforementioned salt is a salt with basic amino acid.

(23) The food of (22), wherein the basic amino acid is selected from the group consisting of arginine, lysine and ornithine.

(24) The food of (22), wherein the basic amino acid is arginine.

(25) The food of (19), wherein the aforementioned compound is an arginine salt with glutamic acid.

(26) The food of any one of (19) to (25), wherein the aforementioned functional gastrointestinal disorder is upper gastrointestinal dysfunction.

(27) The food of (26), wherein the aforementioned upper gastrointestinal dysfunction is functional dyspepsia or gastroesophageal reflux disease.

(28) The food of any one of (19) to (27), wherein a daily dose of the aforementioned compound to an adult is 0.01g - 20 g.

(29) The food of any one of (15) to (28), wherein the content of the aforementioned compound is 0.01 wt% to 10 wt%.

(30) The food of (29), which is a food with health claims or a dietary supplement.

(31) The food of (30), wherein the aforementioned food with health claims is a food for specified health uses or a food with nutrient function claims.

(32) A commercial package comprising a composition comprising at least one kind selected from the group consisting of glutamic acid, 5'-nucleotide and a salt thereof, and a written matter stating that the composition can or should be used for at least one kind selected from prophylaxis or improvement of a functional gastrointestinal disorder, promotion of gastrointestinal motility function and prophylaxis or improvement of dysphagia.

(33) A commercial package comprising a composition comprising at least one kind selected from the group consisting of glutamic acid, 5'-nucleotide and a salt thereof, and a written matter stating that the composition can or should be used for promoting release of NO and/or serotonin specific to the gastrointestinal tract.

(34) The commercial package of (33), wherein the aforementioned gastrointestinal tract is stomach.

(35) A commercial package comprising a food comprising at least one kind of compound selected from the group consisting of glutamic acid, 5'-nucleotide and a salt thereof, and a written matter associated therewith, the written matter stating that the food can or should be used for at least one kind selected from prophylaxis or improvement of a functional gastrointestinal disorder, promotion of gastrointestinal motility function and prophylaxis or improvement of dysphagia.

(36) A commercial package comprising a food comprising at least one kind of compound selected from the group consisting of glutamic acid, 5'-nucleotide and a salt thereof, and a written matter associated therewith, the written matter stating that the food can or should be used for promoting release of NO and/or serotonin specific to the gastrointestinal tract.

(37) The commercial package of (36), wherein the aforementioned gastrointestinal tract is stomach.

(38) Use of glutamic acid, 5'-nucleotide or a salt thereof for the production of an agent for the prophylaxis or improvement of a functional gastrointestinal disorder, which comprises, as an active ingredient, at least one kind selected from the group consisting of glutamic acid, 5'-nucleotide and a salt thereof.

(39) The use of (38), wherein the aforementioned salt is a salt with basic amino acid.

(40) The use of (39), wherein the basic amino acid is selected from the group consisting of arginine, lysine and ornithine.

(41) The use of (39), wherein the basic amino acid is arginine.

(42) The use of (38), wherein the aforementioned active ingredient is an arginine salt with glutamic acid.

(43) The use of any one of (38) to (42), wherein the aforementioned functional gastrointestinal disorder is upper gastrointestinal dysfunction.

(44) The use of (43), wherein the aforementioned upper gastrointestinal dysfunction is functional dyspepsia or gastroesophageal reflux disease.

(45) The use of any one of (38) to (44), wherein a daily dose of the aforementioned active ingredient to an adult is 0.01 g to 20 g.

(46) Use of glutamic acid, 5'-nucleotide or a salt thereof for the production of an agent for the promotion of gastrointestinal motility function, which comprises, as an active ingredient, at least one kind selected from the group consisting of glutamic acid, 5'-nucleotide and a salt thereof.

(47) Use of glutamic acid, 5'-nucleotide or a salt thereof for the production of an agent for the prophylaxis or improvement of dysphagia, which comprises, as an active ingredient, at least one kind selected from the group consisting of glutamic acid, 5'-nucleotide and a salt thereof.

(48) Use of glutamic acid, 5'-nucleotide or a salt thereof for the production of an NO and/or serotonin release promoter in the gastrointestinal tract, which comprises, as an active ingredient, at least one kind selected from the group consisting of glutamic acid, 5'-nucleotide and a salt thereof.

(49) The use of (48), wherein the aforementioned gastrointestinal tract is stomach.

(50) Use of glutamic acid, 5'-nucleotide or a salt thereof for the production of a food for promoting gastrointestinal motility function, which comprises at least one kind of compound selected from the group consisting of glutamic acid, 5'-nucleotide and a salt thereof.

(51) Use of glutamic acid, 5'-nucleotide or a salt thereof for the production of a food for the prophylaxis or improvement of dysphagia, which comprises at least one kind of compound selected from the group consisting of glutamic acid, 5'-nucleotide and a salt thereof.

(52) Use of glutamic acid, 5'-nucleotide or a salt thereof for the production of a food for promoting release of NO and/or serotonin specific to the gastrointestinal tract, which comprises at least one kind of compound selected from the group consisting of glutamic acid, 5'-nucleotide and a salt thereof.

(53) The use of (52), wherein the aforementioned gastrointestinal tract is stomach.

(54) Use of glutamic acid, 5'-nucleotide or a salt thereof for the production of a food for the prophylaxis or improvement of a functional gastrointestinal disorder, which comprises at least one kind of compound selected from the group consisting of glutamic acid, 5'-nucleotide and a salt thereof.

(55) The use of (54), wherein the aforementioned salt is a salt with basic amino acid.

(56) The use of (55), wherein the basic amino acid is selected from the group consisting of arginine, lysine and ornithine.

(57) The use of (55), wherein the basic amino acid is arginine.

(58) The use of (54), wherein the aforementioned active_ ingredient is an arginine salt with glutamic acid.

(59) The use of any one of (54) to (58), wherein the aforementioned functional gastrointestinal disorder is upper gastrointestinal dysfunction.

(60) The use of (59), wherein the aforementioned upper gastrointestinal dysfunction is functional dyspepsia or gastroesophageal reflux disease.

(61) The use of any one of (54) to (60), wherein the daily dose of the aforementioned compound to an adult is 0.01 g - 20 g.

(62) The use of any one of (54) to (61), wherein the content of the aforementioned compound is 0.01 wt% to 10 wt%.

(63) The use of (62), wherein the food is a food with health claims or a dietary supplement.

(64) The use of (63), wherein the aforementioned food with health claims is a food for specified health uses or a food with nutrient function claims.

(65) A method for the prophylaxis or improvement of a functional gastrointestinal disorder, which comprises administering an effective amount of at least one kind selected from the group consisting of glutamic acid, 5'-nucleotide and a salt thereof to a subject of administration.

(66) The method of (65), wherein the aforementioned salt is a salt with basic amino acid.

(67) The method of (66), wherein the basic amino acid is selected from the group consisting of arginine, lysine and ornithine.

(68) The method of (66), wherein the basic amino acid is arginine.

(69) The method of (65), which comprises administering an effective amount of an arginine salt with glutamic acid.

(70) The method of any one of (65) to (69), wherein the aforementioned functional gastrointestinal disorder is upper gastrointestinal dysfunction.

(71) The method of (70), wherein the aforementioned upper gastrointestinal dysfunction is functional dyspepsia or gastroesophageal reflux disease.

(72) The method of any one of (65) to (71), wherein the effective amount to an adult is 0.01 g to 20 g.

(73) A method of promoting gastrointestinal motility function, which comprises administering an effective amount of at least one kind selected from the group consisting of glutamic acid, 5'-nucleotide and a salt thereof to a subject of administration.

(74) A method for the prophylaxis or improvement of dysphagia, which comprises administering an effective amount of at least one kind selected from the group consisting of glutamic acid, 5'-nucleotide and a salt thereof to a subject of administration.

(75) A method for promoting an NO and/or serotonin release in the gastrointestinal tract, which comprises administering an effective amount of at least one kind selected from the group consisting of glutamic acid, 5'-nucleotide and a salt thereof to a subject of administration.

(76) The method of (75), wherein the aforementioned gastrointestinal tract is stomach.

(77) A method for promoting gastrointestinal motility function, which comprises taking a food comprising at least one kind of compound selected from the group consisting of glutamic acid, 5'-nucleotide and a salt thereof.

(78) A method for the prophylaxis or improvement of dysphagia, which comprises taking a food comprising at least one kind of compound selected from the group consisting of glutamic acid, 5'-nucleotide and a salt thereof.

(79) A method for promoting an NO and/or serotonin release in the gastrointestinal tract, which comprises taking a food comprising at least one kind of compound selected from the group consisting of glutamic acid, 5'-nucleotide and a salt thereof.

(80) The method of (79), wherein the aforementioned gastrointestinal tract is stomach.

(81) A method for the prophylaxis or improvement of a functional gastrointestinal disorder, which comprises taking a food comprising at least one kind of compound selected from the group consisting of glutamic acid, 5'-nucleotide and a salt thereof.

(82) The method of (81), wherein the aforementioned salt is a salt with basic amino acid.

(83) The method of (82), wherein the basic amino acid is selected from the group consisting of arginine, lysine and ornithine.

(84) The method of (82), wherein the basic amino acid is arginine.

(85) The method of (81), wherein the aforementioned compound is an arginine salt with glutamic acid.

(86) The method of any one of (81) to (85), wherein the aforementioned functional gastrointestinal disorder is upper gastrointestinal dysfunction.

(87) The method of (86), wherein the aforementioned upper gastrointestinal dysfunction is functional dyspepsia or gastroesophageal reflux disease.

(88) The method of any one of (81) to (87), which comprises taking a food wherein a daily intake of the aforementioned compound to an adult is 0.01 g to 20 g.

(89) The method of any one of (77) to (88), which comprises taking a food wherein the content of the aforementioned compound is 0.01 wt% to 10 wt%.

(90) The method of (89), wherein the food is a food with health claims or a dietary supplement.

(91) The method of (90), wherein the aforementioned food with health claims is a food for specified health uses or a food with nutrient function claims.

**Brief Description of the Drawings**

Fig. 1 shows one example of the effect of a 5-HT3 antagonist against a vagus nerve gastric branches afferent activity induced by MSG (monosodium glutamate) or GMP (sodium guanylate) aqueous solution.

Fig. 2 shows one example of the study results of the activation of vagus nerve by intragastric administration of monosodium glutamate when mucous membrane serotonin is depleted or NO synthesis is inhibited.

Fig. 3 shows one example of the study results of NO release in the mucous membrane by intragastric administration of glutamic acid.

Fig. 4 shows one example of the study results of serotonin leakage in the portal blood by intragastric administration of glutamic acid.

Fig. 5 shows one example of the study results of promotion of stomach emptying by intragastric administration of glutamic acid.

Fig. 6 shows one example of the study results of promotion of stomach emptying by monosodium glutamate and arginine glutamic acid salt.

Fig. 7 shows one example of the study results of promotion of stomach emptying by lysine glutamic acid salt, calcium glutamate and inosinic acid.

Fig. 8 shows one example of the study results of the influence of glutamic acid on the feeling after eating, based on the tension of stomach.

**Best Mode for Embodying the Invention**

The embodiment of the present invention is explained in the following.

As used herein, the "functional gastrointestinal disorder" refers to a pathology where organic diseases such as peptic ulcer and cancer symptoms are not observed, but upper abdominal indefinite complaint continues such as feeling of fullness in the abdomen, nausea, vomiting, upper abdominal pain, anorexia, abnormal bowel movement, and the like, based on the retention of contents in gastrointestinal tract, particularly the stomach. It means a condition without organic disease of the gastrointestinal tract, but with a reproducible gastrointestinal symptom that degrades QOL of patients. The "gastrointestinal tract" in the present invention refers to a series of luminal organs involved in digestion from mouth cavity to anus and, for example, pharynx, esophagus, stomach, small intestine (duodenum, jejunum, ileum) and large intestine can be mentioned. The "upper gastrointestinal tract" refers to pharynx, esophagus, stomach and duodenum.

As used herein, the "functional dyspepsia" refers to a pathology where organic diseases such as peptic ulcer and cancer symptoms are not observed, but upper abdominal indefinite complaint continues such as feeling of fullness in the abdomen, nausea, vomiting, upper abdominal pain, anorexia, abnormal bowel movement and the like, based on the retention of the contents in the stomach. It means a condition without organic disease of the gastrointestinal tract, but with a reproducible gastrointestinal symptom that degrades QOL of patients. The dyspepsia includes diseases so far diagnosed as chronic gastritis and gastritis, and often shows symptoms of abdominal pain, heavy stomach, heartburn and the like. In recent years, 40-60% of the outpatients of medical practitioners is said to suffer from functional dyspepsia, and Helicobacter pylori removal therapy tends to increase the number of functional dyspepsia.

Furthermore, the "gastroesophageal reflux disease" includes reflux esophagitis and is developed by reflux of gastric acid and shows specific symptoms of heartburn, flow up of gastric acid to the mouth and the like. Moreover, while "swallowing" means gulping water and food, it is closely related to not only mouth cavity and pharynx, but also motility of gastrointestinal tract such as esophagus and the like, as evidenced by misswallowing and vomiting due to sticking of swallowed food bolus in the esophagus.

An agent for the prophylaxis or improvement of a functional gastrointestinal disorder, an agent for the promotion of gastrointestinal motility function and an agent for the prophylaxis or improvement of dysphagia are agents for the prophylaxis or improvement to improve upper gastrointestinal dysfunction such as functional gastrointestinal disorders with reproducibility of lowering QOL of patients, particularly functional dyspepsia, gastroesophageal reflux disease and the like.

In the following, the agent for the prophylaxis or improvement of a functional gastrointestinal disorder, the specific promoter of nitric oxide and/or serotonin release in the gastrointestinal tract, the agent for the promotion of gastrointestinal motility function and the agent for the prophylaxis or improvement of dysphagia of the present invention are sometimes simply referred to as a "prophylactic or improving agent".

The prophylactic or improving agent of the present invention contains at least one kind selected from glutamic acid, 5'-nucleotide and a salt thereof as an active ingredient. As 5'-nucleotide, for example, 5'-inosinic acid, 5'-guanylic acid, 5'-adenyl acid, 5'-cytidylic acid, 5'-uridylic acid and 5'-xanthylic acid can be mentioned, of which 5'-inosinic acid and 5'-guanylic acid are preferable.

As the "salt", a pharmacologically acceptable salt of glutamic acid or 5'-nucleotide is preferable. As such salt, salts with inorganic base, salts with inorganic acid, salts with organic acid and salts with organic base and the like can be mentioned. As the salt with inorganic base, alkali metal salts such as sodium, potassium, lithium and the like, alkaline earth metal salts such as calcium, magnesium and the like, ammonium salt and the like can be mentioned. As the salt with inorganic acid, salts with hydrohalic acid (hydrochloric acid, hydrobromic acid, hydrogen iodide acid etc.), sulfuric acid, nitric acid, phosphoric acid and the like can be mentioned. As the salt with organic acid, salts with formic acid, acetic acid, propionic acid, oxalic acid, succinic acid, maleic acid, fumaric acid, citric acid, glutamic acid, aspartic acid, histidine and the like can be mentioned. As the salt with organic base, basic amino acrid (arginine, lysine, ornithine and the like), nucleotide (purine derivative, pyrimidine derivative and the like), alkaloid and the like can be mentioned. Of these, salts with basic amino acid such as arginine and the like, alkali metal salt such as sodium salt and the like, and calcium salt are preferable, salts with organic acid such as inosinic acid, histidine and the like are also useful.

Glutamic acid, 5'-nucleotide and salts thereof to be used may be natural ones derived from animal or plant, or those obtained by a chemical synthetic method, a fermentation method or gene recombination.
As the glutamic acid, an L form, a D form or a mixture thereof (e.g., racemate) can be mentioned, with preference given to an L form. Thus, aspartic acid, tricolominic acid, ibotenic acid and a salt thereof, which are amino acids similar to glutamic acid, are assumed to have an action to improve functional gastrointestinal disorders.

As a preferable active ingredient, glutamic acid, 5'-nucleotide and a salt thereof such as glutamic acid, sodium L-glutamate, sodium D-glutamate, 5'-guanylic acid, sodium 5'-guanylate, sodium 5'-xanthylate, sodium 5'-adenylate, sodium deoxy-5'-adenylate, sodium 5'-inosinate, sodium 2-methylthio-5'-inosinate, sodium N'-methyl-2-methylthio-5'-inosinate and the like can be mentioned. Of these, glutamic acid, sodium L-glutamate and sodium 5'-inosinate are preferable. As the active ingredient, a mixture of one or more kinds thereof can be used.

In the present invention, for example, the improvable specific indefinite complaint in the functional gastrointestinal disorders include, but not limited to, representative upper gastrointestinal indefinite complaint such as nausea, vomiting, sickly feeling, heartburn, feeling of fullness in the abdomen, heavy stomach, belching, chest writhing, chest pain, gastric discomfort, anorexia, dysphagia and the like, lower gastrointestinal indefinite complaint such as abdominal pain, constipation, diarrhea and the like, and-related complaint such as breathlessness, feeling of smothering, low incentive, pharyngeal obstruction feeling of foreign substance ("*baikakuki*" in Chinese medicine), easy fatigability, stiff neck, myotonia, mouth dryness (dry mouth thirst), tachypnea, burning sensation cold sensation of extremities, difficulty in concentration, impatience, sleep disorder, headache, general malaise, palpitation, night sweat, anxiety, dizziness, vertigo, burning sensation, hot flash, sweating, abdominal pain, constipation, depression and the like.

In the present invention, as mentioned above, functional gastrointestinal disorders and the like can be improved by administering an effective amount of the above-mentioned active ingredient to an administration subject. In this case, the active ingredient can be administered orally, enterally or parenterally as it is or after mixing with a pharmaceutical carrier and in the form of a pharmaceutical preparation such as tablet (including sugar-coated tablet, film-coated tablet), pill, capsule, ampoule, divided powder, elixir, suspension, syrup, gum preparation, drop preparation, powder, injection, suppository, sustained-release preparation and the like, in consideration of the amount of an active ingredient to be administered, condition of administration subject (e.g., patient) and the like. As the administration method, oral administration is preferable, and a sustained-release drug is more preferable. As the sustained-release form, conventional sustained-release preparations such as gel-coated preparation, multi-coated preparation and the like, gum preparation, drop preparation, localized release agent (pyloric part rupture preparation) and the like can be mentioned.

As used herein, the "subject of administration" includes individuals affected with functional gastrointestinal disorders and the like (e.g., human, domestic animals and poultry such as bovine, horse, swine, sheep, dog, bird and the like, and experimental animals such as mouse, rat and the like, hereinafter the same), individuals having a risk of being affected with a functional gastrointestinal disorder and the like, and the like. The "effective amount" means an amount sufficient to afford a desired improvement effect. While the dose of the active ingredient varies depending on the sex, age and body weight of administration subject, diet, form of administration, condition of FD and the like, the level of risk inducing FD and the like, condition of organic disease of the gastrointestinal tract and the like, for example, the daily dose of the active ingredient to an adult (body weight 60 kg) is preferably 0.01 - 20 g, more preferably 0.01 - 10 g, and still more preferably 0.1 - 10 g. Such dose can be administered at once or in several portions.

The aforementioned "pharmaceutical carrier" means one which is pharmaceutically acceptable and least causes pharmacological action in the body. As a pharmaceutical carrier for oral administration, binders such as gum tragacanth, gum arabic, cornstarch, gelatin and the like; excipients such as dicalcium phosphate and the like; disintegrants such as cornstarch, potato starch, alginic acid and the like; lubricants such as magnesium stearate and the like; sweetening agents such as sucrose and the like; dyes; flavorings such as orange flavor and the like; solvents such as water, ethanol, glycerol and the like; nutrients such as protein, amino acid, vitamin, lipid, glucose and the like; and the like can be used as appropriate. Furthermore, as a pharmaceutical carrier, pharmaceutically acceptable antioxidants such as cysteine, glutathione, ascorbic acid, sodium metasulfite, sodium bisulfite and the like, and acid neutralizers such as calcium carbonate, hydroxide aluminum gel, aluminum silicate and the like can be used.

The aforementioned dosage forms of the pharmaceutical preparations and pharmaceutical carriers are well known to those of ordinary skill in the art and, for example, the dosage forms and pharmaceutical carriers described in Reimington's Pharmaceutical Science, ed. 16 (1980) and Mack Publishing Company can be used.

The present invention may be used in combination with other pharmaceutical agents, and as such pharmaceutical agents, for example, acid secretion inhibitors such as H2 receptor antagonist, proton pump inhibitor and the like, motility function improvers such as 5-HT receptor agonist, D2 antagonist and the like, antacid agents such as muscarine receptor antagonist, anti-gastrin drug, anticholinergic drug and the like, mucous membrane protectors such as teprenone, plaunotol, ornoprostil, enprostil, misoprostol, rebamipide, sucralfate, polaprezinc, azulene, egualen sodium, glutamine, aldioxa, gefarnate, ecabet sodium and the like, inflammatory colitis treating agents such as sulfasalazine, 5-ASA preparation, steroid, remicade and the like can be contained. One or more kinds of these can be contained. One or more kinds thereof can be contained.

The food of the present invention is now explained. The food of the present invention comprises at least one kind of compound selected from glutamic acid, 5'-nuclebtide and a salt thereof, and is taken for a particular purpose of promotion of specific release of nitric oxide and/or serotonin in the gastrointestinal tract, improvement of functional gastrointestinal disorder, enhancement of gastrointestinal motility function and improvement of dysphagia. In addition, the food of the present invention may be prepared into a common food including what is called a health food. In addition, the food of the present invention may be prepared into a food with health claims, Food for specified health uses, Food with nutrient function claims, and further, a dietary supplement as defined by the food with health claims system of the Ministry of Health; Labour and Welfare. In this case, one or more kinds of glutamic acid, 5'-nucleotide and a salt thereof can be mixed and used.

As the food of the present invention, the aforementioned compound may be taken as it is. For easy intake, however, general food materials, seasonings, flavoring agents and the like may be added to the above-mentioned compound and the mixture is processed into a drink, gum, powder, tablet, granule, jelly and the like before intake. In this case, for example, a tablet made of the above-mentioned compound and a disintegrant, a mixture of the above-mentioned compound and a weighting agent (protein hydrolysate, starch, casein, glucose etc.), a mixture of the above-mentioned compound and an adhesive (gum, sublingual tablet, troche) which permits intraoral sustained-release, a solution of the above-mentioned compound in a solvent capable of dissolving the compound (e.g., edible fat and oil, ethanol, water), a W/O or O/W emulsion containing the above-mentioned compound, or a mixture of the above-mentioned compound and a nutrient (e.g., protein, amino acid, vitamin, lipid, glucose etc.) can be afforded. In addition, at least one kind selected from glutamic acid, 5'-nucleotide and a salt thereof, which is for the prophylaxis or improvement of functional gastrointestinal disorder, enhancement of gastrointestinal motility, prophylaxis or improvement of dysphagia, and promotion of specific release of nitric oxide and/or serotonin in the gastrointestinal tract of the present invention can also be taken with a meal by addition thereto during the meal. For example, they can be taken by addition to an existing food such as drink, soft drink, yogurt, jelly, milk drink and the like.

When the food of the present invention is used for the aforementioned particular object, the amount of intake of glutamic acid, 5'-nucleotide or a salt thereof per day for an adult is preferably 0.01 - 20 g, more preferably 0.01 - 10 g, and still more preferably 0.1 - 10 g. The content of the above-mentioned compound in the food of the present invention is generally 0.001 - 20 wt%, preferably 0.001 - 10 wt%, more preferably 0.01 - 10 wt%, still more preferably 0.1 - 10 wt%. By setting the content of the above-mentioned compound in the above-mentioned common food to fall within the above-mentioned range, a remarkable effect of improving gastrointestinal function can be afforded.

As is clear from the aforementioned explanation, the composition of the present invention may be modified in the aspects clear to those of ordinary skill in the art. Such modifications made therein without departing from the spirit of the invention are also encompassed within the range of the present invention.

**Examples**

The present invention is more specifically explained in the following by referring to Examples, which are not to be construed as limitative.

(Example 1)

To study the effect of a 5-HT3 antagonist on the vagus nerve gastric branches afferent activity induced by an aqueous solution of monosodium glutamate or sodium guanylate, the following experiments (A) - (C) were performed.

SD (IGS) rats (male, 8- to 10-week-old: CHARLES RIVER LABORATORIES JAPAN, INC.) were use for the experiment. After fasting for 15-17 hr, the rats were subjected to laparotomy under urethane anesthesia (1 g/kg, i.p.) to expose ventral vagus nerve gastric branches, and the afferent activity was recorded according to the method (Niijima A., et al., Physiol Behav. 1991 May; 49(5): 1025-8.). An aqueous solution of monosodium glutamate (MSG; 150 mM) or sodium guanylate (GMP; 10, 30, 60 mM) was administered at a rate of 2 mL/rat from the catheter dwelled in the stomach. A 5-HT3 antagonist (granisetron) was administered at a rate of 0.1-10 μg/kg/rat from the catheter dwelled in the femoral vein.

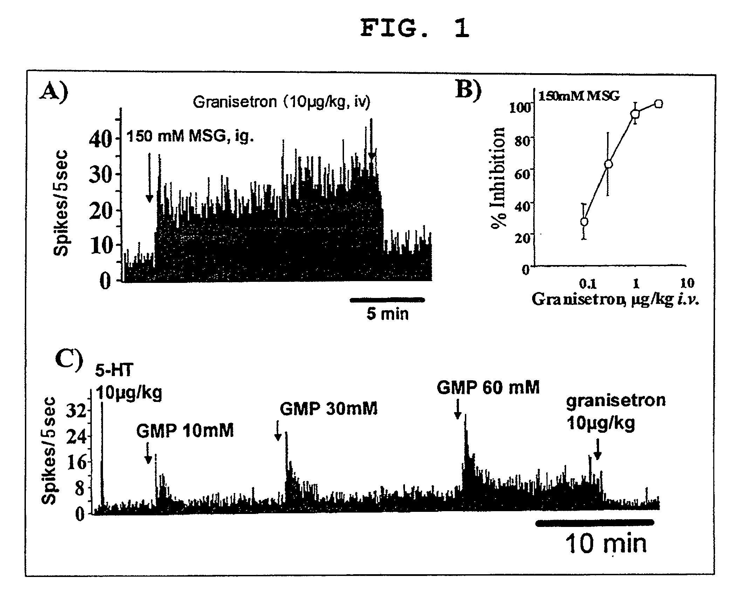

(A) The suppressive effect of granisetron on 150 mM MSG response was studied. The results are shown in Fig. 1A. In Fig. 1A, the vertical axis shows an average nerve fiber spike number for 5 sec, and the axis of abscissas shows time (min).

(B) The dose dependency of the suppressive effect of granisetron on 150 mM MSG response was studied. The results are shown in Fig. 1B. Each data shows mean±standard error of 4 cases.

(C) The dose dependency of GMP response and antagonistic action of granisetron were studied. The results are shown in Fig. 1C. In Fig. 1C, the vertical axis shows an average nerve fiber spike number for 5 sec, and the axis of abscissas shows time (min).

From the results of Fig. 1A, it has been confirmed that vagus nerve gastric branches afferent activity caused by an intragastric administration of MSG aqueous solution is almost completely inhibited by an intravenous administration of granisetron (10 μg/kg), which is a 5-HT3 receptor antagonist. From the results of Fig. 1B, the dose (ED50 value) of granisetron that inhibits half the MSG response was found to be about 0.3 μg/kg/rat. From the results of Fig. 1C, moreover, it has been confirmed that an intragastric administration of GMP (10, 30, 60 mM) aqueous solution dose-dependently activates vagus nerve gastric branches and, like MSG, vagus nerve gastric branches afferent activity induced by GMP is also inhibited by an intravenous administration of granisetron (10 μg/kg).

From the above results, it has been clarified that an intake of monosodium glutamate or sodium guanylate causes release of serotonin in the stomach mucous membrane, and activates vagus nerve via 5-HT3 receptor at the terminal of the vagus nerve gastric branches.

(Example 2)

To study the activation of vagus nerve by intragastric administration of monosodium glutamate when mucous membrane serotonin was depleted or NO synthesis was inhibited, the following experiments (A) and (B) were performed.

SD (IGS) rats (male, 8- to 10-week-old: CHARLES RIVER LABORATORIES JAPAN, INC.) were use for the experiment. After fasting for 15-17 hr, the rats were subjected to laparotomy under urethane anesthesia (1 g/kg, i.p.) to expose ventral vagus nerve gastric branches, and the afferent activity was recorded according to the method (Niijima A., et al., Physiol Behav. 1991 May; 49(5): 1025-8.).

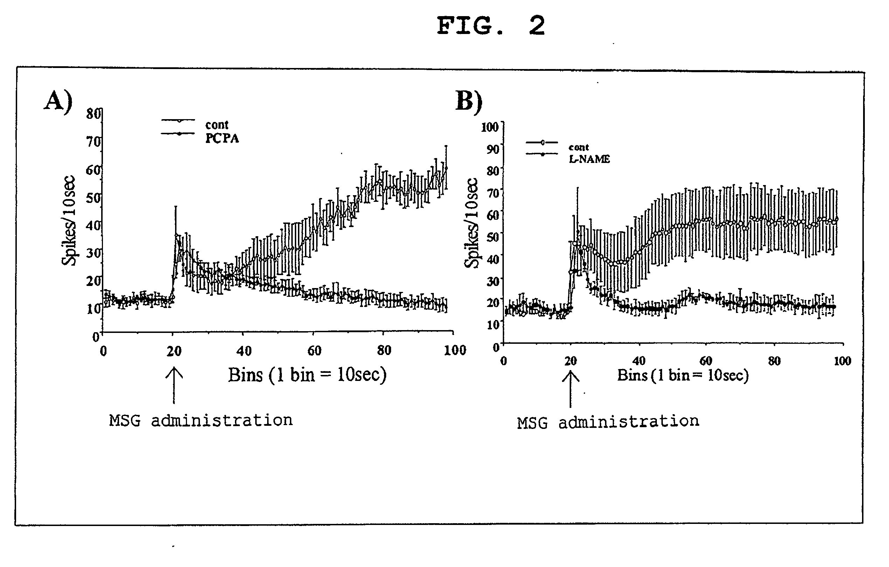

(A) P-chlorophenylalanine (PCPA) was dissolved in 5% CMC solution, and administered at 200 mg/kg/rat twice a day for 2 days (intraperitoneal administration). The final administration of PCPA was performed 15 min before the administration of MSG aqueous solution (150 mM; intragastric administration). The results are shown in Fig. 2A.

(B) NG-nitro-L-arginine methyl ester (L-NAME) was dissolved in saline, and administered at a rate of 10 mg/kg/rat from the cannula dwelled in the femoral vein 15 min before administration of MSG aqueous solution. The results are shown in Fig. 2B.

In Figs. 2A and 2B, the vertical axis shows the average nerve fiber spike number for 10 sec, and the axis of abscissas shows time (1 bin=10 sec). Each data point shows mean±standard error of 4 cases.

From the results of Fig. 2A, it has been confirmed that a pretreatment with PCPA, which is a serotonin synthase inhibitor, eliminates sustained enhancement of nervous activity after intragastric administration of MSG. From the results of Fig. 2B, the same phenomenon as in (A) was confirmed by the pretreatment with L-NAME, which is an NO synthase inhibitor. From these results, it has been clarified that the production of mucous membrane serotonin and NO is essential for the activation of vagus nerve when MSG aqueous solution is intragastrically administered.

(Example 3)

To study NO release in the mucous membrane by an intragastric administration of glutamic acid, the following experiment was performed.

SD rat was subjected to laparotomy with urethane anesthesia, a small incision was made in the anterior stomach and the small intestine, a 2 mm diameter polyethylene tube was inserted, and the perfusate was introduced and discharged with a Perista Pump. The perfusate was heated to 38°C and the flow rate was 1 mL/min. The NO electrode was maintained under the lamina muscularis mucosae together with a temperature sensor, saline was perfused in the stomach for 2-3 hr, the released NO value was stabilized, and isotonic MSG solution (150 mM) was perfused during the time zone of 14:00-18:00.

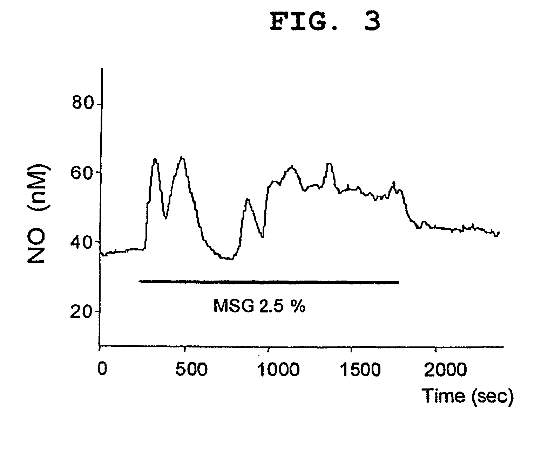

In 3 cases out of 20 cases, an increase in the released NO by isotonic MSG (2.5%) was observed for a latent time of 15 min or so. Representative examples are shown in Fig. 3. In Fig. 3, the vertical axis shows the release NO concentration nM, and the axis of abscissas shows time (sec). When MSG (2.5%) is administered to the stomach mucous membrane by perfusation, NO can be detected by the NO electrode placed on the surface of the stomach mucous membrane. As a result, NO concentration in the stomach mucous membrane was confirmed to increase by MSG intake.

(Example 4)

To study the leakage of serotonin in the portal blood by intragastric administration of glutamic acid, the following experiment was performed.

Male SD rats (8-week-old) were used. They were bred in a light-dark control room with a 7:00-19:00 light period. After fasting at night, the rats were subjected to laparotomy under urethane (1.25 g/kg i.p.) anesthesia, and a catheter for drawing blood was inserted into the portal. The catheter was filled with saline containing 10 unit/mL heparin to prevent hematological coagulation in the catheter. Saline and MSG 450 mM solution were each administered orally by 2 mL using an oral sonde. The blood was drawn every 10 min from 10 min before the administration to 60 min after the administration. To prevent unnecessary platelet activation, blood samples were collected with sufficient attention. The blood drawn was rapidly centrifuged by a conventional method at 4°C or below, 3000 rpm for 15 min to separate the plasma. To the separated plasma was rapidly added an equivalent amount of a mixture (adjusted to pH 2) of acetic acid and hydrochloric acid to eliminate protein. The serotonin (5-HT) amount and 5HIAA amount contained in the supernatant were analyzed by an electric chemical detector (ECD-100, manufactured by Accom Inc.). The analysis conditions were as follows.

| | |
|---|---|
| analysis column | EICOMPAK SC50DS, |
| mobile phase | 83% 0.1M-citric acid .0.IM sodium acetate pH 3.9, |
| | 17% methanol, |
| | 140 mg/L 1-octanesulfonic acid sodium salt (SOS), |
| | 5 mg/L EDTA·2Na, |
| flow rate | 0.23 mL/min, |
| analysis temperature set detector | 25°C, |
| applied potential | +700 mV, |
| active electrode | graphite electrode |

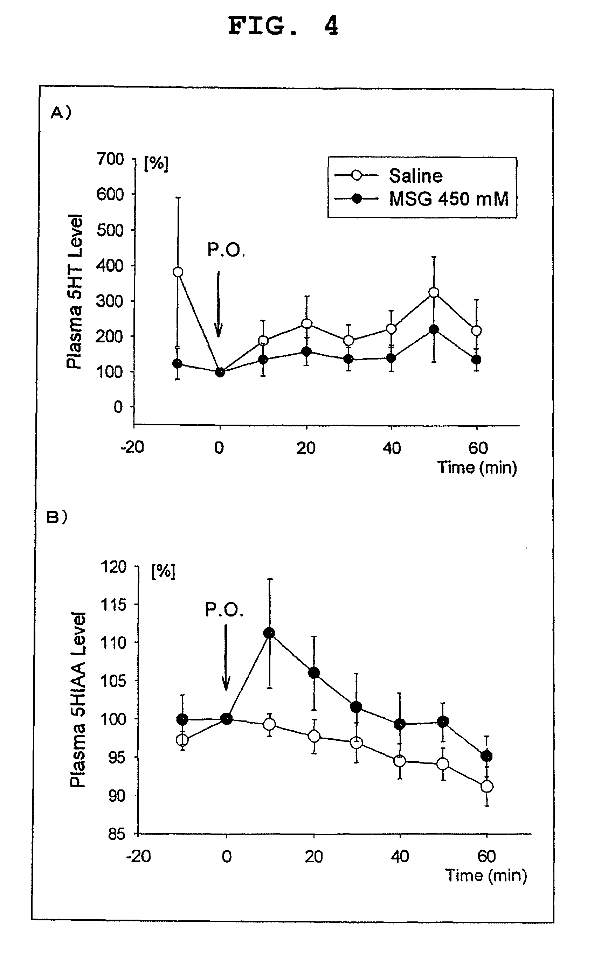

Using the aforementioned analysis method, serotonin and 5HIAA of stable metabolite thereof of the same sample were quantitated. The basic value of plasma 5HT was 29.4±15.7 nM (mean±S.D.). In addition, the basic value of metabolite 5HIAA was 149±22.8 nM. Each was normalized based on the value at 0 min immediately after administration as 100%. The blood collection time immediately after 2 mL intragastric administration was taken as 0 min. The experimental results are shown in Fig. 4. In Fig. 4, the vertical axis shows the relative concentrations of plasma 5HT or 5HIAA, and the axis of abscissas shows time (min). In the results of Fig. 4A, no difference was found between 450 mM MSG administration shown by "• (black circle)", and saline administration shown by "○ (open circle)". From the results of Fig. 4B, however, by the administration of MSG 450 mM, metabolite 5HIAA showed an increase beyond the increase by the saline administration group. Therefrom, it has been clarified that serotonin produced in the stomach mucous membrane by MSG taken is detoxified in the stomach mucous membrane and released as metabolite 5HIAA in the portal. In other words, MSG was confirmed to increase serotonin concentration in the circumscribed area of stomach mucous membrane.

(Example 5)

To study promotion of stomach emptying by intragastric administration of glutamic acid, the following experiment was performed.

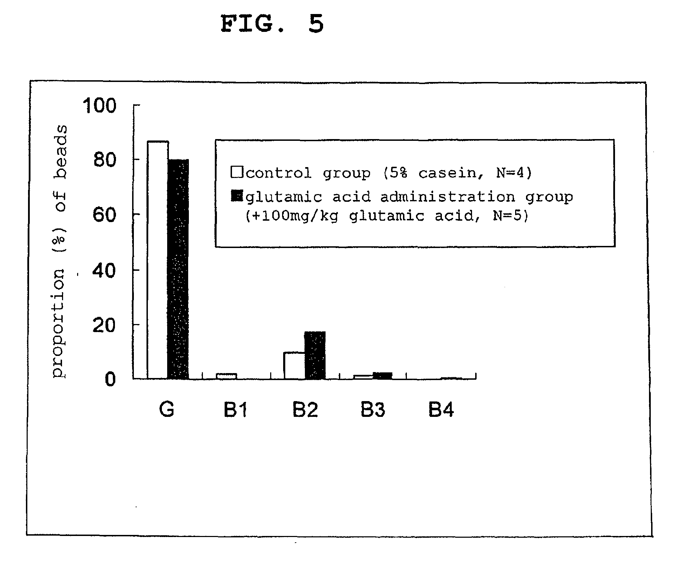

7- to 9-week-old male SD rats were fasted overnight and applied to the experiment. A test diet was orally administered at a volume of 10 mL/kg and, 1 hr later, about 80 glass beads having a diameter of 1 mm were orally administered. The rats were autopsied 30 min later, and the number of beads present in the stomach and small intestine was counted. The small intestine was divided into 4, and named B1-B4 from the area near the stomach. The experimental results are shown in Fig. 5. In Fig. 5, G in the axis of abscissas shows the stomach, and the open column shows the control group. The test diet for the control group was 5% casein dissolved in distilled water, and the test diet for the glutamic acid administration group was 1% monosodium glutamate dissolved in 5% casein (glutamine dose 100 mg/kg). The data shows the proportion in percentage of beads present in the area relative to the total number of beads present in the stomach and small intestine as 100.

From the experiment results, it has been observed that the glutamic acid administration group (N=5) tends to contain a smaller number of beads in the stomach and a large number of beads in the small intestine, as compared to the control group (N=4). Therefrom, it has been confirmed that glutamic acid has a stomach emptying action

(Example 6)

To study promotion of stomach emptying by monosodium glutamate and other salts, the following experiment was performed.

Male ICR mice were used. A 5% casein fluid diet (0.5 mL) containing 0.05% phenol red and a test drug was orally administered, and 30 min later, the chest was opened and the stomach was isolated. The stomach was placed in 0.1N sodium hydroxide (14 mL), homogenized and left standing for 1 hr at room temperature. 20% Trichloroacetic acid (0.5 mL) was added to 5 mL of the supernatant and the mixture was centrifuged (3000 rpm, 20 min). 0.5N sodium hydroxide (4 mL) was added to the supernatant and the absorbance was measured with an absorption spectrometer (560 nm). The gastric emptying rate was determined by the following calculation formula.

$$\text{Gastric emptying rate (\%)} = (1 - \text{absorbance of test sample}/\text{absorbance of standard sample}) \times 100$$

For absorbance of standard sample, the stomach isolated immediately after administration of 0.5% phenol red solution was used.

The test was performed using, after one-way analysis of variance, Dunnett's multiple comparison. *$P<0.05$, **$P<0.01$, ***$P<0.001$.

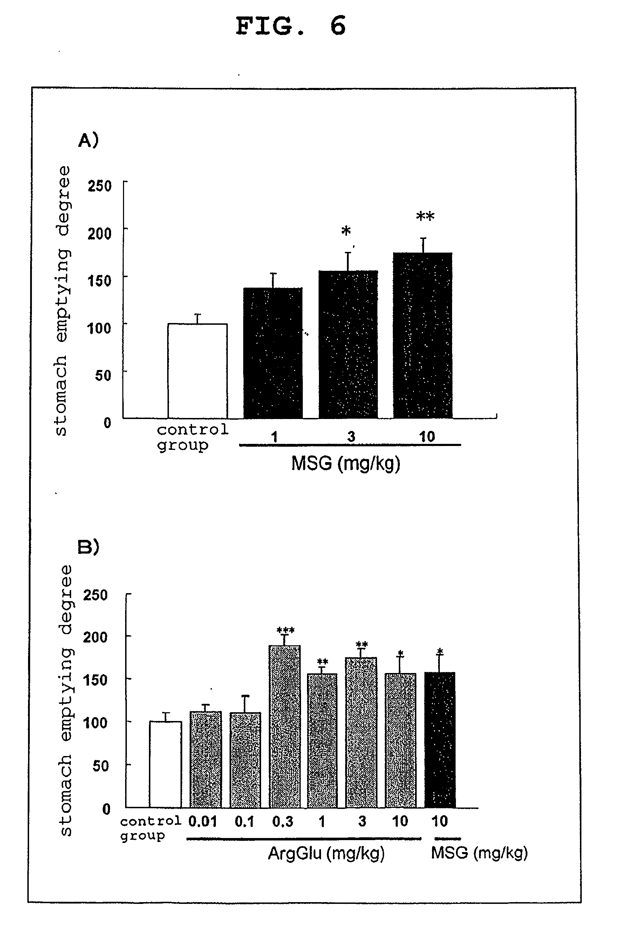

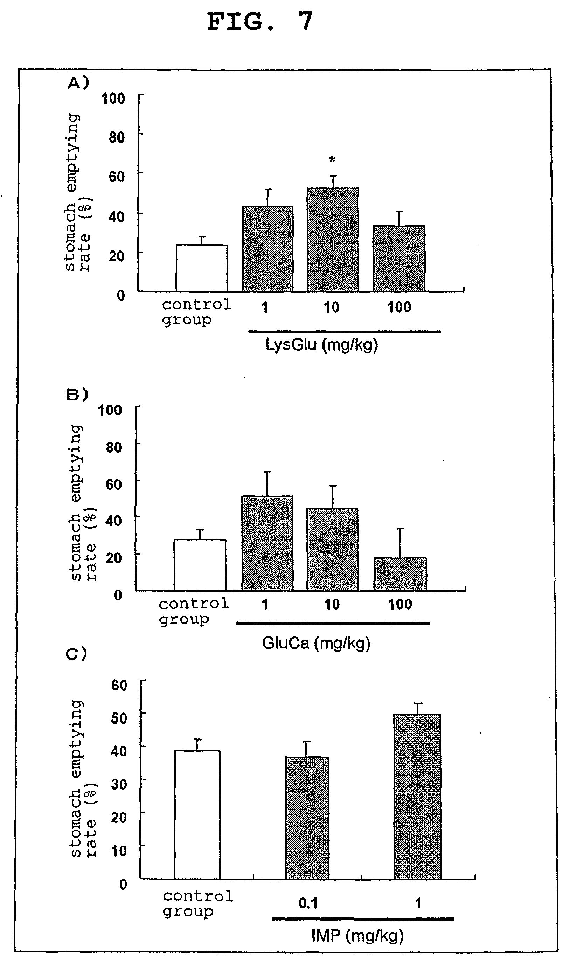

The results are shown in the Figure. The number of cases in each group was 8-24. The vertical axis shows a stomach emptying rate (Figs. 7A, 7B, 7C), and Figs. 6A, 6B show stomach emptying degrees with the stomach emptying rate of the control group as 100. Monosodium glutamate and arginine glutamic acid salt promoted stomach emptying (Figs. 6A, 6B). By comparison of the effects, it has been clarified that arginine salt promotes stomach emptying from lower doses as compared to sodium salt. In addition, lysine glutamic acid salt and calcium glutamate were similarly studied. As a result, it has been clarified that they promote stomach emptying (Figs. 7A, 7B). Based thereon, glutamic acid has been clarified to promote stomach emptying even when it is in other salt form. Moreover, inosinic acid was also studied and found to promote stomach emptying (Fig. 7C).

(Example 7)

To study the effect of monosodium glutamate on feeling after eating, the following experiment was performed.

A double-blind crossover test was performed with 18 healthy males (test subjects) of 45 years old or older. The test subjects drank a casein protein fluid diet (400 mL) within 2 min, and thereafter recorded a score 0-10 on the stomach tension based on the following criteria every 15 min for 4 hr. 0 means no stomach tension, 10 means considerably high stomach tension, and a higher numerical value means higher stomach tension. The test was performed twice, where the test subject drank a test diet containing 0.5% MSG for one time and a control diet for the other time. The composition of the casein protein fluid diet was as follows.

test diet:

| | |
|---|---|
| MSG (Ajinomoto Co., Inc.) | 2.1 g |
| casein calcium (trade name EM9-N: DMV Japan) | 57.96 g |
| dextrin (trade name TK16: Matsutani Chemical) | 52.5 g |
| aspartame (Ajinomoto Co., Inc.) | 0.097 g |
| plum flavor (GIV010790: Givaudan Japan K.K.) | 1.47 g |
| distilled water | 400 mL |
| control diet: | The above-mentioned composition without containing MSG. |

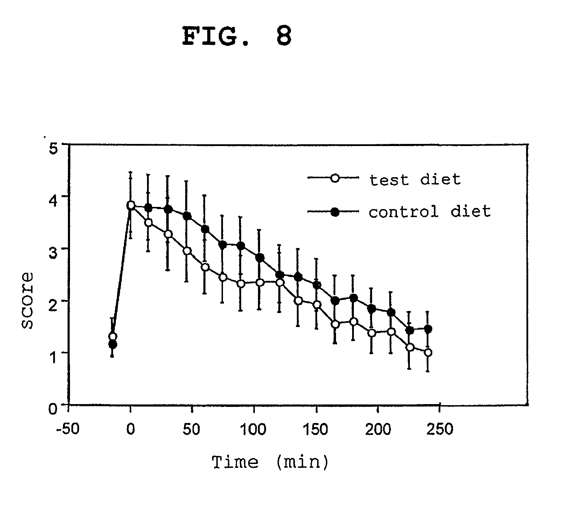

The results are shown in Fig. 8. The time point when the fluid diet was taken was 0 min. In the test diet group, stomach tension was smaller than in the control group. From the above, it has been suggested that glutamic acid improves the feeling after eating.

**Industrial Applicability**

According to the present invention, a pharmaceutical agent and a food useful for the improvement of functional gastrointestinal disorders, particularly upper gastrointestinal dysfunction such as functional dyspepsia, gastroesophageal reflux disease and the like can be provided. Since the concentration of NO and/or serotonin can be increased only in the gastrointestinal tract by the administration of the pharmaceutical agent of the present invention to an administration subject, gastrointestinal motility function can be effectively enhanced. Therefore, indefinite complaint accompanying gastrointestinal dysfunction such as FD and the like can be improved safely and effectively without inducing a systemic side effect heretofore concerned.

While some of the embodiments of the present invention have been described in detail in the above, it will, however, be evident for those of ordinary skill in the art that various modifications and changes may be made to the particular embodiments shown without substantially departing from the teaching and advantages of the present invention. Accordingly, such modifications and changes are encompassed in the spirit and scope of the present invention as set forth in the appended claims.

This application is based on application No. 2004-271884 (filing date: September 17, 2004) filed in Japan, the contents of which are incorporated hereinto by reference.

**Claims**

1.    An agent which comprises, as an active ingredient, a 5'-nucleotide or a salt thereof for use:

in the prophylaxis or improvement of a functional gastrointestinal disorder;
in promotion of gastrointestinal motility function;
in the prophylaxis or improvement of dysphagia; or
in promotion, specific to the gastrointestinal tract, of NO and/or serotonin release.

2.    The agent of claim 1, wherein said 5'-nucleotide is selected from the group consisting of 5'-inosinic acid, 5'-guanylic acid, 5'-adenyl acid, 5'-cytidylic acid, 5'-uridylic acid and 5'-xanthylic acid.

3.    The agent of claim 1, wherein said 5'-nucleotide is selected from 5'-inosinic acid and 5'-guanylic acid.

4.    The agent of any one of claims 1 to 3, wherein said salt is a salt with basic amino acid.

5.    The agent of claim 4, wherein the basic amino acid is selected from the group consisting of arginine, lysine and ornithine.

6.    The agent of claim 4, wherein the basic amino acid is arginine.

**7.** The agent of any one of claims 1 to 6, wherein said functional gastrointestinal disorder is upper gastrointestinal dysfunction.

**8.** The agent of claim 7, wherein said upper gastrointestinal dysfunction is functional dyspepsia or gastroesophageal reflux disease.

**9.** The agent of any one of claims 1 to 8, wherein a daily dose of the active ingredient to an adult is 0.01 g to 20 g.

**10.** The agent of claim 1, for use in promotion, specific to the stomach, of NO and/or serotonin release.

**11.** An agent according to any one of claims 1 to 10 which is a food.

**12.** The agent of claim 11, wherein the content of said compound is 0.01 wt% to 10 wt%.

**13.** Use of a 5'-nucleotide or a salt thereof for the production of an agent of any one of claims 1 to 12.

FIG. 1

FIG. 2

A)

B)

MSG administration

MSG administration

EP 2 305 241 A1

# FIG. 3

# FIG. 4

FIG. 5

# FIG. 6

# FIG. 7

# FIG. 8

Europäisches
Patentamt

European
Patent Office

Office européen
des brevets

**EUROPEAN SEARCH REPORT**

Application Number

EP 10 01 2576

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 1 767 201 A (AJINOMOTO KK [JP]) 28 March 2007 (2007-03-28) * paragraph [0001] * * paragraphs [0010], [0015], [0028] * * paragraph [0017] * * paragraph [0007] * ----- | 1-13 | INV. A61K31/198 A61K31/7068 A61K31/7072 A61K31/7076 A61K31/708 A61P1/04 |
| X | EP 0 218 453 A (PFIZER [US]) 15 April 1987 (1987-04-15) * the whole document * * page 2, line 46; claims 4-6 * ----- | 1-13 | A61P1/14 A61P43/00 A23L1/30 A23L1/305 |

TECHNICAL FIELDS
SEARCHED      (IPC)

A61K
A23L
A61P

The present search report has been drawn up for all claims

| Place of search | Date of completion of the search | Examiner |
|---|---|---|
| Munich | 21 January 2011 | Ansaldo, M |

EPO FORM 1503 03.82 (P04C01)

**ANNEX TO THE EUROPEAN SEARCH REPORT
ON EUROPEAN PATENT APPLICATION NO.**

EP 10 01 2576

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2011

| Patent document cited in search report | | Publication date | Patent family member(s) | | Publication date |
|---|---|---|---|---|---|
| EP 1767201 | A | 28-03-2007 | WO | 2006001492 A1 | 05-01-2006 |
| | | | KR | 20070039083 A | 11-04-2007 |
| | | | US | 2007218109 A1 | 20-09-2007 |
| EP 0218453 | A | 15-04-1987 | AU | 563822 B2 | 23-07-1987 |
| | | | AU | 6342186 A | 09-04-1987 |
| | | | CA | 1273875 A1 | 11-09-1990 |
| | | | DE | 3667914 D1 | 08-02-1990 |
| | | | DK | 468486 A | 03-04-1987 |
| | | | GR | 3000305 T3 | 07-06-1991 |
| | | | HU | 46216 A2 | 28-10-1988 |
| | | | IE | 58635 B1 | 20-10-1993 |
| | | | JP | 1782467 C | 13-08-1993 |
| | | | JP | 4070285 B | 10-11-1992 |
| | | | JP | 62096424 A | 02-05-1987 |
| | | | NZ | 217778 A | 29-03-1989 |
| | | | PT | 83472 A | 01-11-1986 |
| | | | US | 4994457 A | 19-02-1991 |
| | | | ZA | 8607494 A | 25-05-1988 |

EPO FORM P0459

For more details about this annex : see Official Journal of the European Patent Office, No. 12/82

**EP 2 305 241 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 4133366 B
- US 2003138476 A
- EP 0318446 B
- JP 56057385 A
- CA 2404005 B
- JP 48030583 A
- JP 2004271884 A

**Non-patent literature cited in the description**

- **Elia M ; Lunn PG.** *Nutrition,* July 1997, vol. 13 (7-8), 743-7
- **Vasilevskaia LS et al.** *Vopr Pitan.,* May 1993, 29-33
- **Rymshina MV ; Vasilevskaia LS.** *Vopr Pitan.,* 1996, 9-11
- **Kochetkov AM.** *Vopr Pitan.,* September 1992, 19-22
- **Shlygin GK.** *Klin Med (Mosk).,* August 1991, vol. 69 (8), 66-70
- **PJ. Reeds ; DG Burin ; B Stoll ; Jahoor.** *J. Nutrition,* 2000, vol. 130, 978
- **Niijima A. et al.** *Physiol Behav.,* May 1991, vol. 49 (5), 1025-8
- **Niijima A. et al.** *J Nutr.,* April 2000, vol. 130 (4S), 9715-3
- Reimington's Pharmaceutical Science. Mack Publishing Company, 1980